# EUROPEAN PATENT APPLICATION

(11) **EP 4 732 931 A1**
(43) Date of publication of application: **29.04.2026**
(21) Application number: 24882358.5
(22) Date of filing: 21.10.2024
(51) Int. Cl.: B01D 53/047, B01D 53/14, B01J 20/06, C01B 32/50, C07C 1/22, C07C 9/04, C07C 29/15, C07C 31/04

(54) **METHOD FOR SEPARATING AND RECOVERING CARBON DIOXIDE AND METHOD FOR PRODUCING METHANOL**

(30) Priority: 26.10.2023 JP 2023184157
(71) Applicant: JFE Steel Corporation, Tokyo, 100-0011 (JP)
(72) Inventor: YOSHIKAWA, Kohei, Tokyo 100-0011 (JP); SHIGAKI, Nobuyuki, Tokyo 100-0011 (JP); NISHIKAWA, Yuta, Tokyo 100-0011 (JP)
(74) Representative: Hoffmann Eitle
(86) International application number: PCT/JP2024/037456
(87) International publication number: WO 2025/089245

(57) **Abstract**

A method of carbon dioxide separation and recovery that can separate and recover carbon dioxide with hydrogen sulfide and carbonyl sulfide removed from a feed gas containing carbon dioxide, hydrogen sulfide, and carbonyl sulfide. This method of carbon dioxide separation and recovery from a feed gas G01 containing carbon dioxide, hydrogen sulfide, and carbonyl sulfide by pressure swing adsorption includes a dehumidification process of removing moisture from the feed gas G01 to produce a dehumidified feed gas, and a carbon dioxide recovery process of separating and recovering carbon dioxide gas from the dehumidified feed gas by pressure swing adsorption. The method also includes a carbonyl sulfide hydrolysis process of reacting carbonyl sulfide with moisture to hydrolyze into hydrogen sulfide and carbon dioxide, and a desulfurization process of separating the hydrogen sulfide.

## Description

### TECHNICAL FIELD

The present disclosure relates to methods of carbon dioxide separation and recovery and methods of producing methanol.

### BACKGROUND

In recent years, there has been demand to reduce carbon dioxide (CO₂) emissions in order to help prevent global warming. Steelworks emit large amounts of by-product gases, including blast furnace gas, a by-product of blast furnaces, coke oven gas generated from coke ovens, and converter gas generated from converters. Of these, blast furnace gas, which is a particularly large source of emissions, is used as an energy source within steelworks, but blast furnace gas contains a large amount of CO₂ that is emitted as is. Therefore, separating and effectively utilizing CO₂ from blast furnace gas is an important issue.

One method for effectively utilizing CO₂ is to use a catalyst to convert CO₂ into valuable products such as methanol and methane. For example, in Patent Literature (PTL) 1, a method is proposed for synthesizing methanol from a feed gas containing CO₂ and hydrogen (H₂) using a catalyst.

The catalyst used in the synthesis of methanol and methane is deteriorated by sulfur compounds such as hydrogen sulfide (H₂S) and carbonyl sulfide (COS) in the blast furnace gas. Therefore, in order to effectively utilize the CO₂ contained in the blast furnace gas, it is necessary to separate and desulfurize the CO₂ from the blast furnace gas.

As a method for separating CO₂, there is a pressure swing adsorption (PSA) method that utilizes a pressure dependency of an adsorption amount in an adsorbent such as zeolite. For example, in PTL 2, a method is proposed for separating and recovering CO₂ from a feed gas containing CO₂ using a PSA method.

A possible process for separating CO₂ from reducing furnace gas such as blast furnace gas (gas containing CO₂, carbon monoxide (CO), H₂, nitrogen (N₂), water (H₂O), H₂S, and COS) by the PSA method described in PTL 2 and synthesizing methanol using a catalyst is illustrated in FIG. 1.

First, a feed gas G01 such as reducing furnace gas is introduced into a dehumidification apparatus 1 installed upstream of CO₂ separation by a PSA device 2 to dehumidify the feed gas G01 and decrease the H₂O concentration of the feed gas G01. This is because in the PSA method, when the H₂O concentration in the feed gas is high, the CO₂ separation performance is greatly decreased.

Next, the dehumidified feed gas G01 with decreased H₂O concentration is introduced into the PSA device 2. In the PSA device 2, first, in the adsorption process, the dehumidified feed gas G01 is introduced into an adsorption column of the PSA device 2, CO₂ is adsorbed by adsorbent loaded in the adsorption column, and a non-adsorbed gas G02 containing non-adsorbed gas components that were not adsorbed by the adsorbent is discharged.

Subsequently, in the desorption process, pressure inside the adsorption column is decreased by a pump 3A to desorb the CO₂ adsorbed by the adsorbent, and gas containing CO₂ is discharged from the adsorption column as desorbed gas G03.

The desorbed gas G03 that has been discharged is compressed by a compressor 3B, mixed with H₂ gas G04, and sent to a methanol synthesis apparatus 4, where methanol G05 is synthesized according to the reaction of the following expression (1).

CO₂ + 3H₂ → CH₃OH + H₂O (1)

### CITATION LIST

### Patent Literature

PTL 1: JP 7049075 B2
PTL 2: JP 6677181 B2

### SUMMARY

### (Technical Problem)

As described above, blast furnace gas contains, in addition to the main gas components CO₂, CO, and H₂, gas components containing sulfur (S) derived from iron ore and coke, such as H₂S and COS. The H₂S and COS are adsorbed together with CO₂ by the adsorbent in the adsorption column. Therefore, when blast furnace gas is used as the feed gas G01, H₂S and COS are separated and recovered from the feed gas G01 together with CO₂, and there is a problem in that they become impurity components of the CO₂ gas. When the desorbed gas G03 containing such H₂S and COS is introduced into the methanol synthesis apparatus 4 to carry out a methanol synthesis reaction, the catalyst used in the methanol synthesis reaction is deteriorated.

In view of the above-mentioned problem, it would be helpful to provide a method of carbon dioxide separation and recovery that can separate and recover carbon dioxide with hydrogen sulfide and carbonyl sulfide removed, from a feed gas containing carbon dioxide, hydrogen sulfide, and carbonyl sulfide.

### (Solution to Problem)

[1] A method of carbon dioxide separation and recovery from a feed gas containing carbon dioxide, hydrogen sulfide, and carbonyl sulfide by pressure swing adsorption, the method comprising:
   a dehumidification process of removing moisture from the feed gas to obtain a dehumidified feed gas;
   a carbon dioxide recovery process of separating and recovering carbon dioxide gas from the dehumidified feed gas by pressure swing adsorption;
   a carbonyl sulfide hydrolysis process of reacting the carbonyl sulfide with moisture to hydrolyze into hydrogen sulfide and carbon dioxide; and
   a desulfurization process of separating the hydrogen sulfide.
[2] The method of carbon dioxide separation and recovery according to [1], wherein the carbonyl sulfide hydrolysis process is carried out before the dehumidification process.
[3] The method of carbon dioxide separation and recovery according to [2], wherein the desulfurization process is carried out after the carbon dioxide recovery process.
[4] The method of carbon dioxide separation and recovery according to [2], wherein the desulfurization process is carried out after the carbonyl sulfide hydrolysis process, before the carbon dioxide recovery process.
[5] The method of carbon dioxide separation and recovery according to [1], wherein the carbonyl sulfide hydrolysis process is carried out after the carbon dioxide recovery process.
[6] The method of carbon dioxide separation and recovery according to [5], further comprising a humidity adjustment process of adjusting moisture content of the carbon dioxide gas after the carbon dioxide recovery process, before the carbonyl sulfide hydrolysis process.
[7] The method of carbon dioxide separation and recovery according to [6], wherein the humidity adjustment process is a water washing process of washing the carbon dioxide gas with water.
[8] The method of carbon dioxide separation and recovery according to [6], wherein the humidity adjustment process is a water vapor addition process of adding water vapor to the carbon dioxide gas.
[9] The method of carbon dioxide separation and recovery according to any one of [1] to [8], wherein a catalyst used in the carbonyl sulfide hydrolysis process is a catalyst containing an oxide of aluminum.
[10] The method of carbon dioxide separation and recovery according to any one of [1] to [8], wherein a catalyst used in the carbonyl sulfide hydrolysis process is a catalyst containing an oxide or sulfide of cobalt, or an oxide or sulfide of molybdenum, or both an oxide or sulfide of cobalt and an oxide or sulfide of molybdenum.
[11] The method of carbon dioxide separation and recovery according to any one of [1] to [10], wherein a desulfurizing agent used in the desulfurization process is a dry desulfurizing agent, and the dry desulfurizing agent contains at least one selected from the group consisting of oxides of iron, oxides of copper, and oxides of zinc.
[12] The method of carbon dioxide separation and recovery according to [11], wherein at least two types of dry desulfurizing agents are used in the desulfurization process, at least one type of dry desulfurizing agent contains an oxide of iron, and at least one type of dry desulfurizing agent contains an oxide of copper.
[13] The method of carbon dioxide separation and recovery according to any one of [1] to [12], further comprising a compression process of pressurizing the carbon dioxide gas to obtain a compressed gas, and subjecting the compressed gas to the desulfurization process.
[14] The method of carbon dioxide separation and recovery according to any one of [1] to [13], wherein the feed gas is a reducing furnace gas.
[15] The method of carbon dioxide separation and recovery according to [14], wherein the reducing furnace gas is a blast furnace gas.
[16] A method of producing methanol, the method comprising synthesizing a hydrocarbon or methanol from carbon dioxide obtained by using the method of carbon dioxide separation and recovery according to any one of [1] to [15].

### (Advantageous Effect)

According to the present disclosure, it is possible to separate and recover carbon dioxide from which hydrogen sulfide and carbonyl sulfide have been removed, from a feed gas containing carbon dioxide, hydrogen sulfide, and carbonyl sulfide.

### BRIEF DESCRIPTION OF THE DRAWINGS

In the accompanying drawings:
FIG. 1 is a diagram illustrating a flowchart of an example of a conventional method of carbon dioxide separation and recovery;
FIG. 2 is a diagram illustrating a flowchart of a preferred example of a method of carbon dioxide separation and recovery according to the present disclosure; and
FIG. 3 is a diagram illustrating a flowchart of a preferred example of a method of producing methanol according to the present disclosure.

### DETAILED DESCRIPTION

An embodiment of the present disclosure is described below with reference to the drawings. The method of carbon dioxide separation and recovery according to the present disclosure is a method of separating and capturing carbon dioxide from a feed gas containing carbon dioxide, hydrogen sulfide, and carbonyl sulfide by pressure swing adsorption, and includes a dehumidification process of removing moisture from the feed gas to obtain a dehumidified feed gas, and a carbon dioxide recovery process of separating and capturing carbon dioxide gas from the dehumidified feed gas by pressure swing adsorption. Here, the method is characterized by comprising a carbonyl sulfide hydrolysis process of reacting carbonyl sulfide with moisture to hydrolyze into hydrogen sulfide and carbon dioxide, and a desulfurization process of separating the hydrogen sulfide.

The present inventors have conducted intensive studies into a method for separating and recovering CO₂ with H₂S and COS removed from a feed gas containing CO₂, H₂S, and COS. As a result, the inventors arrived at the idea of converting COS, which is relatively difficult to remove, into H₂S by pre-hydrolysis as illustrated in the following expression (2), and then desulfurizing the obtained H₂S together with the H₂S originally contained in the feed gas. This allows CO₂ with H₂S and COS removed to be obtained from the feed gas, and when the obtained CO₂ gas is introduced into the methanol synthesis apparatus 4 to synthesize methanol, deterioration of the catalyst used in the methanol synthesis reaction can be suppressed.

COS + H₂O → CO₂ + H₂S (2)

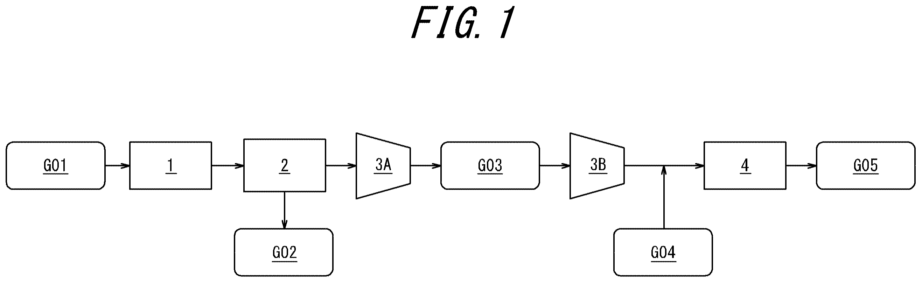

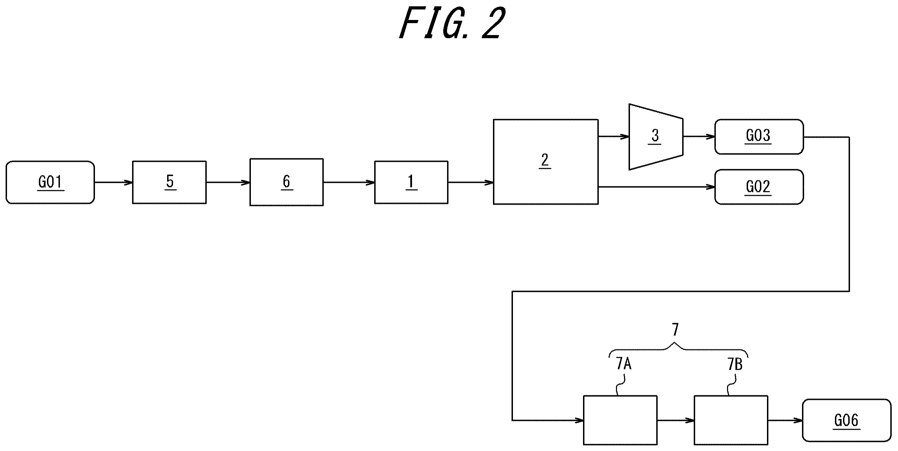

FIG. 2 illustrates a flowchart of a preferred example of the method of carbon dioxide separation and recovery according to the present disclosure. In FIG. 2, the same components as those illustrated in FIG. 1 are denoted by the same reference signs. The method of carbon dioxide separation and recovery according to the present disclosure is specifically described below using the flowchart illustrated in FIG. 2, but the present disclosure is not limited to this example.

First, the feed gas G01 is introduced into a humidity adjustment apparatus 5, and H₂O concentration of the feed gas G01 is adjusted (humidity adjustment process). The feed gas G01 is a gas containing CO₂, H₂S, and COS. Such gases include, for example, by-product gases emitted from steelworks. Examples of by-product gases include reducing furnace gases containing CO₂, CO, N₂, H₂, H₂S, and COS, such as blast furnace gas, coke oven gas, and converter gas. The disclosed method can effectively remove COS and H₂S from blast furnace gas even from among the above gases, and separate and recover CO₂ gas, thereby reducing CO₂ emissions.

The humidity adjustment apparatus 5 is an apparatus that adjusts the H₂O concentration of the feed gas G01. Such a humidity adjustment apparatus 5 can be configured as a water washing apparatus that washes the feed gas G01 with water. In such a case, the humidity adjustment process is configured as a water washing process. In the water washing process, the H₂O concentration of the feed gas G01 can be adjusted by adjusting the water washing temperature. Further, in a case where the feed gas G01 contains water-soluble gas components such as HCl and NH₃, the water washing process can remove these water-soluble gas components.

The humidity adjustment apparatus 5 can be configured as a water vapor addition apparatus that adds water vapor to the feed gas G01. In such a case, the humidity adjustment process is configured as a water vapor addition process. In the water vapor addition apparatus, the H₂O concentration of the feed gas G01 can be controlled by adjusting the flow rate of water vapor.

Next, the feed gas G01 whose H₂O concentration has been adjusted in the humidity adjustment process is introduced into a COS hydrolysis apparatus 6, where the COS contained in the feed gas G01 is reacted with H₂O according to the above expression (2) and hydrolyzed into H₂S and CO₂ (carbonyl sulfide hydrolysis process).

The COS hydrolysis apparatus 6 can be configured as a catalyst-loaded column loaded with a catalyst that promotes the reaction of the above expression (2). The catalyst that is loaded may be a catalyst containing an oxide of aluminum (Al). The catalyst may contain an oxide or sulfide of cobalt (Co), an oxide or sulfide of molybdenum (Mo), or both.

In this carbonyl sulfide hydrolysis process, the feed gas G01 containing COS and H₂O is passed through the COS hydrolysis apparatus 6, whereby the COS contained in the feed gas G01 can be hydrolyzed. A temperature of the COS hydrolysis apparatus 6 such as a catalyst-loaded column (that is, the temperature of the catalyst in the loaded column) is preferably set to 100 °C or higher, from the viewpoint of helping prevent condensation of H₂O. H₂S is often more reactive with a dry desulfurizing agent than COS, and by converting COS to H₂S, the efficiency of the dry desulfurizing agent is improved.

Subsequently, the feed gas G01 in which COS has been converted to H₂S is introduced into the dehumidification apparatus 1, where moisture is removed from the feed gas G01 to obtain a dehumidified feed gas (dehumidification process).

The dehumidification apparatus 1 can be configured as a dehumidification device that cools the feed gas G01 and condenses H₂O to dehumidify, for example. Further, the dehumidification apparatus 1 can be configured as a desiccant type apparatus such as a loaded adsorption column loaded with an adsorbent that adsorbs H₂O, such as Al oxide, silicon (Si) oxide, or zeolite.

The dehumidification process is preferably carried out until the dew point of the feed gas G01 becomes 0 °C or lower. The dew point more preferably becomes -20 °C or lower. This makes it possible to suppress a decrease in the amount of CO₂ adsorbed by the adsorbent in the subsequent carbon dioxide recovery process.

Thereafter, the dehumidified feed gas G01 obtained in the dehumidification process is introduced into the PSA device 2, and CO₂ gas is separated and recovered from the dehumidified feed gas G01 by the PSA method (carbon dioxide recovery process).

The PSA device 2 can be configured as an adsorption column loaded with an adsorbent that adsorbs CO₂, such as zeolite. In the adsorption process, the dehumidified feed gas G01 is circulated through the adsorption column, whereby CO₂ is adsorbed onto the adsorbent, and non-adsorbed gas components that are not adsorbed onto the adsorbent are discharged from the adsorption column as a non-adsorbed gas G02. Then, in the desorption process, the inside of the adsorption column is depressurized using a pump 3, thereby desorbing CO₂ from the adsorbent, and a desorbed gas G03 containing the desorbed CO₂ is separated and recovered. At this time, gas pressure on an inlet side of the pump 3 is, for example, 1 kPa or more and 20 kPa or less, and gas pressure on an outlet side of the pump 3 is, for example, 100 kPa or more. In this carbon dioxide recovery process, when the feed gas G01 is blast furnace gas, CO, N₂, and H₂ in the feed gas G01 are separated to be main components of the non-adsorbed gas G02, and CO₂, H₂S, COS, and H₂O are separated to be main components of the desorbed gas G03.

The pressure in the adsorption column during decompression in the desorption process is preferably 1 kPa or more. The pressure is preferably 20 kPa or less. The pressure is more preferably 5 kPa or more. The pressure is more preferably 10 kPa or less.

Next, the desorbed gas G03 is introduced into a desulfurization apparatus 7, where H₂S contained in the desorbed gas G03 is separated (desulfurization process).

The desulfurization apparatus 7 can be configured as a desulfurizing agent-loaded column loaded with a dry desulfurizing agent that adsorbs H₂S. By passing the desorbed gas G03 through the desulfurizing agent-loaded column, the H₂S can be adsorbed onto the desulfurizing agent and desulfurized.

Examples of dry desulfurizing agents include oxides of iron (Fe), oxides of copper (Cu), and oxides of zinc (Zn), and a desulfurizing agent containing one or more selected from the group consisting of these oxides can be used.

Further, it is preferable to use at least two types of dry desulfurizing agents, at least one of which contains an oxide of Fe, and at least one of which contains an oxide of Cu. There are various possible combinations of desulfurizing agents and the order in which they are used, but it is preferable to first remove H₂S contained in the desorbed gas G03 using an inexpensive dry desulfurizing agent containing Fe oxide, and then remove remaining H₂S and COS using a dry desulfurizing agent containing Cu oxide.

In the desulfurization process using at least two types of desulfurizing agents, the number of desulfurizing agent-loaded columns is equal to or greater than the number of types of desulfurizing agents, each desulfurizing agent-loaded column is loaded with a desulfurizing agent, and the desorbed gas G03 is sequentially circulated through each desulfurizing agent-loaded column, thereby desulfurizing the desorbed gas G03. For example, when two types of dry desulfurizing agents are used, two desulfurizing agent-loaded columns 7A and 7B are installed as illustrated in FIG. 2, with the desulfurizing agent-loaded column 7A being loaded with a dry desulfurizing agent containing Fe oxide and the desulfurizing agent-loaded column 7B being loaded with a dry desulfurizing agent containing Cu oxide. Then, by sequentially passing the desorbed gas G03 through the desulfurizing agent-loaded columns 7A and 7B, the desorbed gas G03 is desulfurized to obtain CO₂ gas G06, which is the desorbed gas G03 with decreased H₂S concentration and COS concentration.

In the above description, the COS hydrolysis apparatus 6 is provided upstream of the dehumidification apparatus 1, and the carbonyl sulfide hydrolysis process is carried out upstream of the dehumidification process, but the present disclosure is not limited to this. The COS hydrolysis apparatus 6 may be provided downstream of the PSA device 2, and the carbonyl hydrolysis process may be carried out downstream of the carbon dioxide recovery process.

However, in the COS hydrolysis process, it is preferable to use a feed gas G01 in which the H₂O concentration has been increased to 1 % or more. However, the dehumidification apparatus 1 is installed to maintain the performance of the PSA device 2, and there is no apparatus for increasing the H₂O concentration downstream of the dehumidification apparatus 1. Therefore, when the H₂O concentration is low and COS cannot be sufficiently hydrolyzed in the COS hydrolysis process, COS that does not easily react with the desulfurizing agent remains in the gas, and there is a possibility that this will flow into the catalyst in the desulfurization apparatus 7 in the subsequent stage and deteriorate the catalyst. On the other hand, the feed gas G01 after the water washing process has an H₂O concentration close to the vapor pressure at that temperature. For example, when the water washing process is carried out at 25 °C, the H₂O concentration of the feed gas G01 can easily be made 1 % or more. Therefore, the feed gas G01 after the water washing process is suitable for COS hydrolysis. Therefore, as illustrated in FIG. 2, it is preferable to provide the COS hydrolysis apparatus 6 upstream of the dehumidification apparatus 1 and to carry out the carbonyl sulfide hydrolysis process upstream of the dehumidification process.

When the COS hydrolysis apparatus 6 is provided downstream of the PSA apparatus 2 and the carbonyl hydrolysis process is carried out downstream of the carbon dioxide recovery process, it is preferable to further provide the humidity adjustment apparatus 5 downstream of the PSA apparatus 2, upstream of the COS hydrolysis apparatus 6, and to further carry out the humidity adjustment process for adjusting the moisture content of the carbon dioxide gas downstream of the carbon dioxide recovery process, upstream of the carbonyl sulfide hydrolysis process. This allows the COS contained in the desorbed gas G03 to be sufficiently hydrolyzed.

Further, in FIG. 2, the desulfurization apparatus 7 is provided downstream of the PSA device 2, and the desulfurization process is carried out downstream of the carbon dioxide recovery process. This is because the performance of the adsorbent such as zeolite used in the carbon dioxide recovery process is not deteriorated by H₂S and COS, so there is no need to provide the desulfurization apparatus 7 upstream of the PSA device 2. However, it is also possible to provide the desulfurization apparatus 7 upstream of the PSA device 2 and carry out the desulfurization process upstream of the carbon dioxide recovery process. Further, the desulfurization apparatus 7 may be provided upstream of the PSA device 2, downstream of the COS hydrolysis apparatus 6, so that the desulfurization process is carried out downstream of the carbonyl sulfide hydrolysis process, upstream of the carbon dioxide recovery process.

However, from the viewpoint of suppressing carbon deposition (coking) resulting from CO in the gas, a gas with a low CO concentration is preferred, and therefore it is preferable to provide the desulfurization apparatus 7 downstream of the PSA device 2 and carry out the desulfurization process downstream of the carbon dioxide recovery process. Further, adsorbents such as zeolite used in the PSA method also adsorb H₂S and COS. Therefore, by providing the desulfurization apparatus 7 downstream of the PSA device 2, H₂S and COS are concentrated on the side of the desorbed gas G03 containing CO₂ discharged from the PSA device 2. The desorbed gas G03 has higher concentrations of H₂S and COS than the feed gas G01 and flow rate is decreased, which has the effect of increasing the desulfurization capacity and decreasing the amount of H₂S and COS that slips from the outlet of the desulfurization apparatus 7.

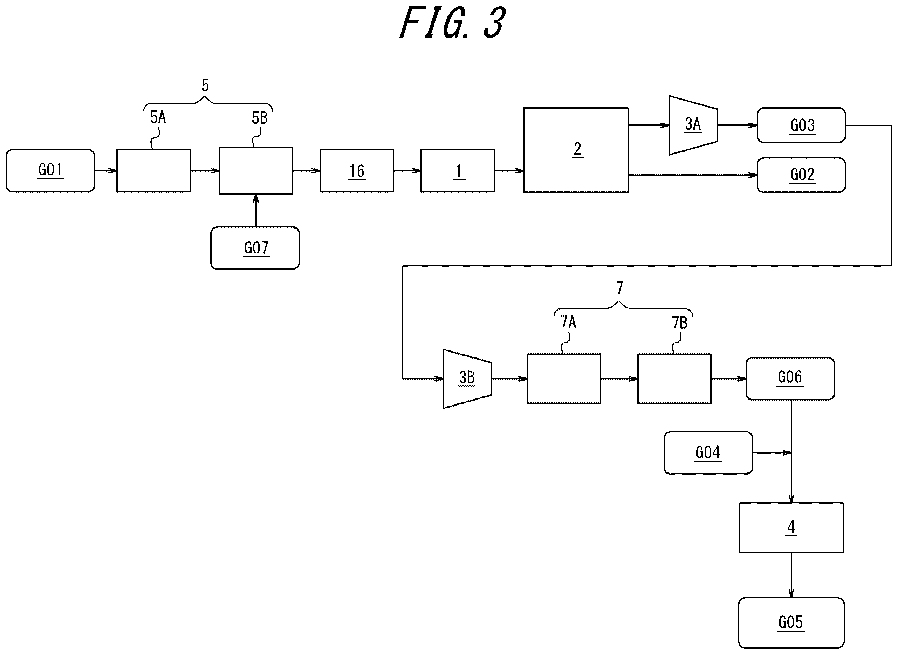

FIG. 3 illustrates a flowchart of a preferred example of the method of producing methanol according to the present disclosure. In FIG. 3, the same components as those illustrated in FIG. 2 are denoted by the same reference signs. The difference between the flowchart illustrated in FIG. 3 and the flowchart illustrated in FIG. 2 is that in the flowchart illustrated in FIG. 3, the configuration carries out a water-gas shift reaction process in addition to the COS hydrolysis process in a COS hydrolysis apparatus 16. Further, in the flowchart illustrated in FIG. 3, the configuration carries out a compression process in which the desorbed gas G03 is introduced into the compressor 3B and compressed to a pressure of 1000 kPa or more to obtain a compressed gas. Further, in the flowchart illustrated in FIG. 3, the CO₂ gas G06 from which H₂S and COS have been removed in the desulfurization process is introduced into a methanol synthesis apparatus 4 to produce methanol G05. The differences are explained below.

First, in order to carry out the water-gas shift reaction process in the COS hydrolysis apparatus 16, the humidity adjustment apparatus 5 is composed of a humidity adjustment apparatus 5A that carries out a water washing process and a humidity adjustment apparatus 5B that carries out a water vapor addition process of adding water vapor G07 to the feed gas G01. First, the feed gas G01 is introduced into the humidity adjustment apparatus 5A and subjected to a water washing process to adjust the H₂O concentration of the feed gas G01. Next, the feed gas G01 with the adjusted H₂O concentration is introduced into the humidity adjustment apparatus 5B, and water vapor is added to the feed gas G01 to increase the H₂O concentration. The feed gas G01 having an increased H₂O concentration is then introduced into the COS hydrolysis apparatus 16, where the carbonyl sulfide hydrolysis process and the water-gas shift reaction process are carried out. This makes it possible to improve the efficiency of CO₂ processing.

In the humidity adjustment apparatus 5B, the water vapor G07 is added to the feed gas G01. From the viewpoint of preventing carbon deposition on the catalyst in the COS hydrolysis apparatus 16, the molar ratio of H₂O/CO in the feed gas G01 after the addition of the water vapor G07 is preferably 1.0 or more. The molar ratio is more preferably 2.0 or more. Further, the water-gas shift reaction is an exothermic reaction and a lower temperature is more advantageous in terms of chemical equilibrium, and therefore the temperature of the catalyst in the COS hydrolysis apparatus 16 is preferably 200 °C or higher. The temperature is preferably 500 °C or lower. The temperature is more preferably 200 °C or higher. The temperature is more preferably 400 °C or lower. Further, the temperature of the feed gas G01 after the addition of the water vapor G07 is preferably 200 °C or higher. The temperature is preferably 500 °C or lower. The temperature is more preferably 200 °C or higher. The temperature is more preferably 400 °C or lower.

Following the COS hydrolysis process and the water-gas shift reaction process, the dehumidification process is carried out in which H₂O is removed from the feed gas G01 in the dehumidification apparatus 1 to obtain the dehumidified feed gas G01. The dehumidification process is preferably carried out so that the dew point of the feed gas G01 becomes 0 °C or lower. The dew point more preferably becomes -20 °C or lower. Thereafter, in the adsorption process, the dehumidified feed gas G01 is passed through the PSA apparatus 2 that includes an adsorbent-loaded column loaded with zeolite as an adsorbent, so that CO₂ is adsorbed onto the adsorbent, and the non-adsorbed gas G02 containing non-adsorbed gas components that were not adsorbed onto the adsorbent is discharged. Then, in the desorption process, the pressure inside the adsorption column is decreased by the pump 3A, CO₂ is desorbed from the adsorbent, and the gas containing CO₂ is discharged from the adsorption column as the desorbed gas G03.

Subsequently, the desorbed gas G03 discharged from the adsorption column is introduced into the compressor 3B and compressed. The pressure of the desorbed gas G03 may be set to an appropriate value depending on a subsequent reaction. In the upstream PSA device 2, when methanol is synthesized, the pressure of the desorbed gas G03 is preferably adjusted via the compressor 3B to 1 MPa or more. The pressure is preferably adjusted to 10 MPa or less.

The desorbed gas G03 is then passed through the desulfurizing agent-loaded column 7A loaded with a dry desulfurizing agent containing Fe oxide, and then passed through the desulfurizing agent-loaded column 7B loaded with a dry desulfurizing agent containing Cu oxide, thereby decreasing the H₂S concentration and the COS concentration of the desorbed gas G03. The desorbed gas G03 having decreased H₂S and COS concentrations is recovered as CO₂ gas G06. The use of the desorbed gas G03 that is pressurized is preferable because it is expected to increase desulfurization capacity and promote the desulfurization reaction. In order to improve the desulfurization performance of the desulfurizing agent-loaded column 7B, a dehumidification process may be carried out by further installing the dehumidification apparatus 1 upstream.

Then, the CO₂ gas G06 is mixed with the H₂ gas G04, and the mixture is introduced into the methanol synthesis apparatus 4, where the methanol G05 is synthesized by the reaction of the above expression (1). The CO₂ gas G06, which is a raw material for the methanol G05, has H₂S and COS removed, so that deterioration of the catalyst used in the synthesis reaction of the methanol G05 can be suppressed.

In the above description, the methanol synthesis apparatus 4 is configured to produce the methanol G05, but a configuration is possible to produce a hydrocarbon. In such a case, instead of the methanol synthesis apparatus 4, a hydrocarbon synthesis apparatus loaded with a hydrocarbon synthesis catalyst may be used. For example, when the hydrocarbon to be synthesized is methane, the hydrocarbon synthesis apparatus is configured to keep a reaction vessel loaded with a catalyst containing Ni at a temperature of 200 °C or higher and 400 °C or lower, and to circulate the CO₂ gas G06 and the H₂ gas G04.

### INDUSTRIAL APPLICABILITY

According to the present disclosure, it is possible to separate and recover carbon dioxide from which hydrogen sulfide and carbonyl sulfide have been removed, from a feed gas containing carbon dioxide, hydrogen sulfide, and carbonyl sulfide.

### REFERENCE SIGNS LIST

- 1: dehumidification apparatus
- 2: pressure swing adsorption device
- 3, 3A: pump
- 3B: compressor
- 4: methanol synthesis apparatus
- 5, 5A, 5B: humidity adjustment apparatus
- 6, 16: carbonyl sulfide hydrolysis apparatus
- 7: desulfurization apparatus
- G01: feed gas
- G02: non-adsorbed gas
- G03: desorbed gas
- G04: hydrogen gas
- G05: methanol
- G06: carbon dioxide gas
- G07: water vapor

## Claims

1. A method of carbon dioxide separation and recovery from a feed gas containing carbon dioxide, hydrogen sulfide, and carbonyl sulfide by pressure swing adsorption, the method comprising:
a dehumidification process of removing moisture from the feed gas to obtain a dehumidified feed gas;
a carbon dioxide recovery process of separating and recovering carbon dioxide gas from the dehumidified feed gas by pressure swing adsorption;
a carbonyl sulfide hydrolysis process of reacting the carbonyl sulfide with moisture to hydrolyze into hydrogen sulfide and carbon dioxide; and
a desulfurization process of separating the hydrogen sulfide.

2. The method of carbon dioxide separation and recovery according to claim 1, wherein the carbonyl sulfide hydrolysis process is carried out before the dehumidification process.

3. The method of carbon dioxide separation and recovery according to claim 2, wherein the desulfurization process is carried out after the carbon dioxide recovery process.

4. The method of carbon dioxide separation and recovery according to claim 2, wherein the desulfurization process is carried out after the carbonyl sulfide hydrolysis process, before the carbon dioxide recovery process.

5. The method of carbon dioxide separation and recovery according to claim 1, wherein the carbonyl sulfide hydrolysis process is carried out after the carbon dioxide recovery process.

6. The method of carbon dioxide separation and recovery according to claim 5, further comprising a humidity adjustment process of adjusting moisture content of the carbon dioxide gas after the carbon dioxide recovery process, before the carbonyl sulfide hydrolysis process.

7. The method of carbon dioxide separation and recovery according to claim 6, wherein the humidity adjustment process is a water washing process of washing the carbon dioxide gas with water.

8. The method of carbon dioxide separation and recovery according to claim 6, wherein the humidity adjustment process is a water vapor addition process of adding water vapor to the carbon dioxide gas.

9. The method of carbon dioxide separation and recovery according to any one of claims 1 to 8, wherein a catalyst used in the carbonyl sulfide hydrolysis process is a catalyst containing an oxide of aluminum.

10. The method of carbon dioxide separation and recovery according to any one of claims 1 to 8, wherein a catalyst used in the carbonyl sulfide hydrolysis process is a catalyst containing an oxide or sulfide of cobalt, or an oxide or sulfide of molybdenum, or both an oxide or sulfide of cobalt and an oxide or sulfide of molybdenum.

11. The method of carbon dioxide separation and recovery according to any one of claims 1 to 10, wherein a desulfurizing agent used in the desulfurization process is a dry desulfurizing agent, and the dry desulfurizing agent contains at least one selected from the group consisting of oxides of iron, oxides of copper, and oxides of zinc.

12. The method of carbon dioxide separation and recovery according to claim 11, wherein at least two types of dry desulfurizing agents are used in the desulfurization process, at least one type of dry desulfurizing agent contains an oxide of iron, and at least one type of dry desulfurizing agent contains an oxide of copper.

13. The method of carbon dioxide separation and recovery according to any one of claims 1 to 12, further comprising a compression process of pressurizing the carbon dioxide gas to obtain a compressed gas, and subjecting the compressed gas to the desulfurization process.

14. The method of carbon dioxide separation and recovery according to any one of claims 1 to 13, wherein the feed gas is a reducing furnace gas.

15. The method of carbon dioxide separation and recovery according to claim 14, wherein the reducing furnace gas is a blast furnace gas.

16. A method of producing methanol, the method comprising synthesizing a hydrocarbon or methanol from carbon dioxide obtained by using the method of carbon dioxide separation and recovery according to any one of claims 1 to 15.
